# EUROPEAN PATENT APPLICATION

(11) **EP 4 406 469 A1**
(43) Date of publication of application: **31.07.2024**
(21) Application number: 22884122.7
(22) Date of filing: 24.10.2022
(51) Int. Cl.: A61B 1/00, A61B 1/04, A61B 34/00

(54) **MAGNETIC FIELD CONTROL DEVICE**

(30) Priority: 22.10.2021 KR 20210142088
(71) Applicant: IUCF-HYU (INDUSTRY-UNIVERSITY COOPERATION FOUNDATION HANYANG UNIVERSITY), Seoul 04763 (KR)
(72) Inventor: JANG, Gun Hee, Seoul 06326 (KR); LEE, Won Seo, Yongin-si Gyeonggi-do 16900 (KR); LEE, Dae Hee, Seoul 04805 (KR); KIM, Seung Uk, Seoul 01309 (KR)
(74) Representative: Prüfer & Partner mbB Patentanwälte · Rechtsanwälte
(86) International application number: PCT/KR2022/016231
(87) International publication number: WO 2023/068901

(57) **Abstract**

A magnetic field control device is disclosed. A magnetic field control device includes: a housing having an accommodation space formed therein; a first magnet member which is positioned in the accommodation space, has a central shaft disposed parallel to a first axis, and generates a magnetic field; a second magnet member which is positioned at a side of the first magnet member in the direction of a second axis perpendicular to the first axis, has a central shaft disposed parallel to the first axis, and generates a magnetic field; a magnet drive part for rotating the first magnet member and the second magnet member around the central shafts thereof, respectively; and a control part for controlling the magnet drive part to adjust angles of the first magnet member and the second magnet member.

## Description

### [Technical Field]

The present invention relates to a magnetic field control device, and more particularly, to a magnetic field control device capable of controlling a posture of a capsule endoscope.

### [Background Art]

A magnetic field control device for actively driving an object in which a permanent magnet is mounted, such as a magnetically driven capsule endoscope and a wired/wireless magnetic robot, which operate within a human body, is being developed. The object in which the permanent magnet is mounted may be driven by receiving a magnetic torque and a magnetic force from an external magnetic field, and may be precisely controlled remotely so that various applications in the medical field and the research and development have been performed. Representative examples may include a magnetically driven capsule endoscope applied to a digestive system, a magnetic catheter applied to a treatment of cardiac arrhythmia, and the like, and there may be a vascular treatment magnetic robot for a vascular occlusion treatment, a microrobot for intraocular drug delivery, a magnetic nanoparticle for targeted drug delivery within tissues, and the like in addition to the above examples.

Among devices for generating external magnetic fields, a device that utilizes an electromagnet may require a power generation device that occupies large volume and weight and entails a large cost in order to apply and control a current to the electromagnet. In addition, a device that utilizes a permanent magnet may require a large mechanical part similar to an industrial robot arm, so that the device has not been distributed in actual medical sites due to limitations such as a space, a cost, and compatibility with peripheral devices.

Accordingly, there is a need to develop a magnetic field control device that is miniaturized, has a simple structure, and allows a medical staff to precisely control an object in which a permanent magnet is mounted when the object is inserted into a body of a patient.

### [Disclosure]

### [Technical Problem]

The present invention provides a magnetic field control device capable of precisely controlling an object in which a permanent magnet is mounted, which is located within a body.

In addition, the present invention provides a magnetic field control device that is miniaturized and has a simple structure.

### [Technical Solution]

According to the present invention, a magnetic field control device includes: a housing having an accommodation space formed in the housing; a first magnet member located in the accommodation space, having a central shaft arranged parallel to a first axis, and configured to generate a magnetic field; a second magnet member located on one side of the first magnet member in a direction of a second axis that is perpendicular to the first axis, having a central shaft arranged parallel to the first axis, and configured to generate a magnetic field; a magnet drive part for rotating the first magnet member and the second magnet member about the central shafts of the first magnet member and the second magnet member, respectively; and a control part for controlling the magnet drive part to adjust angles of the first magnet member and the second magnet member.

In addition, each of the first magnet member and the second magnet member may be a permanent magnet bisected into an N-pole and an S-pole based on the central shafts of the first magnet member and the second magnet member.

In addition, the permanent magnet may have a cylindrical or spherical shape.

In addition, the permanent magnet may have a longitudinal direction arranged in a direction of the first axis.

In addition, the permanent magnet may have a longitudinal direction arranged in a direction of a third axis that is perpendicular to the first axis and the second axis.

In addition, the magnetic fields generated from the first magnet member and the second magnet member, respectively, may have a same intensity.

In addition, each of the first magnet member and the second magnet member may include: cores having a longitudinal direction arranged in a direction of a third axis that is perpendicular to the first axis and the second axis; coils wound around the cores, respectively; and a power supply part for applying currents to the coils, and the control part may control the currents applied to the cores.

In addition, the magnetic field control device may further include: a third magnet member located on an opposite side of the second magnet member in the direction of the second axis, having a central shaft arranged parallel to the first axis, and configured to generate a magnetic field, and the magnet drive part may rotate the third magnet member about the central shaft of the third magnet member.

In addition, the first to third magnet members may be located at a same height.

In addition, the first magnet member and the third magnet member may be located at a higher position than the second magnet member in the direction of the third axis.

In addition, the magnetic field control device may further include: a housing drive part for rotating the housing about a third axis that is perpendicular to the first axis and the second axis.

In addition, the magnetic field control device may further include: a first connection frame fixedly coupled to an upper portion of the housing, and configured to support the housing drive part at a first point; a second connection frame coupled to the first connection frame through a pin at a second point of the first connection frame; and a handle coupled to the pin, and the first connection frame may be capable of performing relative rotation with respect to the second connection frame about the pin.

### [Advantageous Effects]

According to the present invention, angles of at least two magnet members may be adjusted to generate distributions of magnetic fields having various intensities and directions within an operation region, so that an object in which a permanent magnet is mounted, which is located within a body, can be precisely controlled.

In addition, according to the present invention, a housing, magnet members, a magnet drive part, and a housing drive part may be coupled to each other, so that a magnetic field control device can be miniaturized and can have a simple structure.

### [Description of Drawings]

FIG. 1 is a perspective view showing a magnetic field control device according to one embodiment of the present invention.
FIG. 2 is a perspective view showing a magnet member of FIG. 1.
FIG. 3 is a view showing the magnet member of FIG. 2 and a target region.
FIG. 4 is a flowchart showing a process of aligning magnet members according to an embodiment of the present invention.
FIG. 5 is a view showing distributions of magnetic fields generated in an operation region due to various angle changes of the magnet members according to one embodiment of the present invention.
FIG. 6 is a view showing a state in which a capsule endoscope rotates about a third axis direction under control of a magnetic field control device according to an embodiment of the present invention.
FIG. 7 is a view showing a magnetic field control device according to another embodiment of the present invention.
FIG. 8 is a view showing distributions of magnetic fields generated in an operation region due to various angle changes of magnet members according to another embodiment of the present invention.
FIG. 9 is a perspective view showing a magnetic field control device according to still another embodiment of the present invention.
FIG. 10 is a view showing distributions of magnetic fields generated in an operation region due to various angle changes of magnet members according to the embodiment of FIG. 9.
FIG. 11 is a view showing an arrangement of first to third magnet members according to yet another embodiment of the present invention.
FIG. 12 is a view showing a magnetic field control device according to still yet another embodiment of the present invention.
FIG. 13 is a view showing a magnetic field control device according to another embodiment of the present invention.
FIG. 14 is a view showing a magnetic field control device according to still another embodiment of the present invention.

### [Best Mode]

According to an embodiment of the present invention, a magnetic field control device includes: a housing having an accommodation space formed in the housing; a first magnet member located in the accommodation space, having a central shaft arranged parallel to a first axis, and configured to generate a magnetic field; a second magnet member located on one side of the first magnet member in a direction of a second axis that is perpendicular to the first axis, having a central shaft arranged parallel to the first axis, and configured to generate a magnetic field; a magnet drive part for rotating the first magnet member and the second magnet member about the central shafts of the first magnet member and the second magnet member, respectively; and a control part for controlling the magnet drive part to adjust angles of the first magnet member and the second magnet member.

### [Mode for Invention]

Hereinafter, exemplary embodiments of the present invention will be described in detail with reference to the accompanying drawings. However, the technical idea of the present invention is not limited to the embodiments described herein, but may be embodied in different forms. The embodiments introduced herein are provided to sufficiently deliver the idea of the present invention to those skilled in the art so that the disclosed contents may become thorough and complete.

When it is mentioned in the present disclosure that one element is on another element, it means that one element may be directly formed on another element, or a third element may be interposed between one element and another element. Further, in the drawings, thicknesses of films and regions are exaggerated for effective description of the technical contents.

In addition, although the terms such as first, second, and third have been used to describe various elements in various embodiments of the present disclosure, the elements are not limited by the terms. The terms are used only to distinguish one element from another element. Therefore, an element mentioned as a first element in one embodiment may be mentioned as a second element in another embodiment. The embodiments described and illustrated herein include their complementary embodiments, respectively. Further, the term "and/or" used in the present disclosure is used to include at least one of the elements enumerated before and after the term.

As used herein, an expression in a singular form includes a meaning of a plural form unless the context clearly indicates otherwise. Further, the terms such as "including" and "having" are intended to designate the presence of features, numbers, steps, elements, or combinations thereof described in the present disclosure, and shall not be construed to preclude any possibility of the presence or addition of one or more other features, numbers, steps, elements, or combinations thereof. In addition, the term "connection" used herein is used to include both indirect and direct connections of a plurality of elements.

Further, in the following description of the present invention, detailed descriptions of known functions or configurations incorporated herein will be omitted when they may make the gist of the present invention unnecessarily unclear.

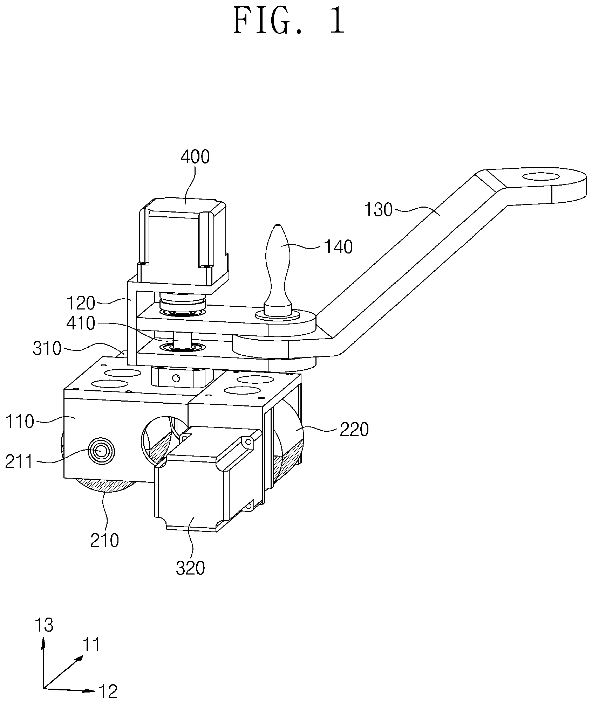

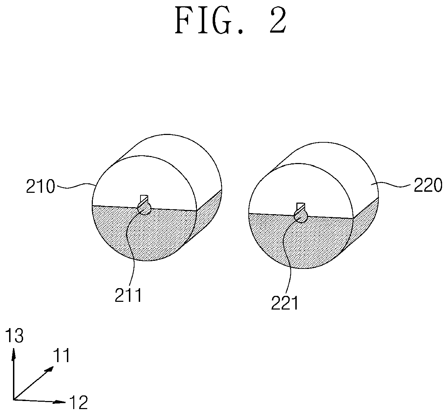

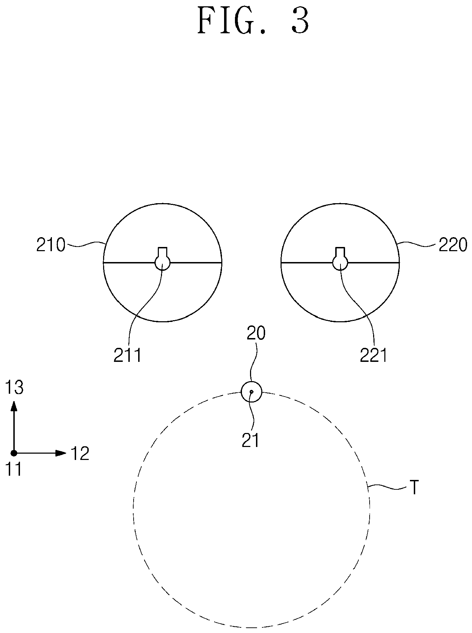

FIG. 1 is a perspective view showing a magnetic field control device according to one embodiment of the present invention, FIG. 2 is a perspective view showing a magnet member of FIG. 1, and FIG. 3 is a view showing the magnet member of FIG. 2 and a target region.

Referring to FIGS. 1 to 3, a magnetic field control device 10 may generate and control a magnetic field and a magnetic force. The magnetic field and the magnetic force may be used to control a movement of an object 20 in which a permanent magnet is mounted, which is located within an operation region T. The object 20 in which the permanent magnet is mounted may be a magnetically driven capsule endoscope that is applicable to a digestive system, or a magnetic catheter that is applicable to a treatment of cardiac arrhythmia. In the present embodiment, the magnetically driven capsule endoscope will be illustrated as the object 20 in which a permanent magnet is mounted.

The capsule endoscope 20 may be inserted into a digestive organ of a human body so as to be used to inspect the digestive organ, and may include a permanent magnet 21 inside the capsule endoscope 20. The capsule endoscope 20 may have a posture controlled in a direction of the magnetic field by a magnetic torque formed by an interaction between the magnetic field generated by the magnetic field control device 10 and a magnetic field of the permanent magnet 21.

The magnetic field control device 10 may include a housing 110, connection members 120 and 130, magnetic field generation parts 210 and 220, magnet drive parts 310 and 320, a housing drive part 400, and a control part (not shown).

The housing 110 may have a predetermined shape, and may support the magnetic field generation parts 210 and 220. According to an embodiment, the housing 110 may have a hexahedral shape, and may be formed therein with an accommodation space having an open bottom.

The connection members 120 and 130 may connect the housing 110 to a support device (not shown) . The support device may be a lamp or a monitor of an operating room, a ceiling or a wall of a medical procedure room, a mobile cart, or the like. The connection members 120 and 130 may include a first connection frame 120 and a second connection frame 130.

The first connection frame 120 may be located at a top of the housing 110. A rotation shaft 410 of the housing drive part 400 may be inserted into a first point of the first connection frame 120.

The second connection frame 130 may have a predetermined length, and may have one end coupled to the first connection frame 120 through a pin at a second point of the first connection frame 120. The first connection frame 120 may perform relative rotation with respect to the second connection frame 130 about the pin. A handle 140 may be provided on a top of the pin. An opposite end of the second connection frame 130 may be coupled to the support device. A user may grip the handle 140 with a hand to control a movement of the magnetic field control device 10. Through a manipulation of the user, the second connection frame 130 may perform relative rotation with respect to the support device, and the first connection frame 120 may perform the relative rotation with respect to the second connection frame 130.

The magnetic field generation parts 210 and 220 may be provided in the accommodation space of the housing 110. The magnetic field generation parts 210 and 220 may include at least two magnet members. Two, three, four, five, or more magnetic flux members may be provided. In the present embodiment, two magnet members 210 and 220 will be illustrated to be provided. First and second magnet members 210 and 220 may generate magnetic fields, respectively. According to an embodiment, the first and second magnet members 210 and 220 may be provided as permanent magnets. Each of first and second permanent magnets 210 and 220 may have a cylindrical shape having a predetermined height, and may have a structure bisected into an N-pole and an S-pole based on central shafts 211 and 221 of the first and second permanent magnets 210 and 220. In addition, the first and second permanent magnets 210 and 220 may have a spherical shape having a predetermined diameter, and may have a structure bisected into an N-pole and an S-pole based on central shafts of the first and second permanent magnets 210 and 220.

The first and second permanent magnets 210 and 220 may have the central shafts 211 and 221 arranged parallel to a first axis direction 11. The first and second permanent magnets 210 and 220 may be spaced apart from each other by a predetermined distance in a second axis direction 12 that is perpendicular to the first axis direction 11. The first and second permanent magnets 210 and 220 may be coupled to the housing 110 so as to be rotatable about the central shafts 211 and 221 of the first and second permanent magnets 210 and 220, respectively. According to one embodiment, the first and second permanent magnets 210 and 220 may be arranged at the same height in a third axis direction 13. According to another embodiment, the first and second permanent magnets 210 and 220 may be arranged at different heights in the third axis direction 13.

The magnet drive parts 310 and 320 may individually rotate the first and second permanent magnets 210 and 220 about the central shafts 211 and 221 of the first and second permanent magnets 210 and 220. The magnet drive parts 310 and 320 may rotate the first and second permanent magnets 210 and 220 by using electricity or a hydraulic pressure. In the present embodiment, a motor driven by electricity will be illustrated to be used as each of the magnet drive parts 310 and 320.

The magnet drive parts 310 and 320 may include a first lower motor 310 and a second lower motor 320. The first lower motor 310 may be coupled to the central shaft 211 of the first permanent magnet 210, and may rotate the first permanent magnet 210. The second lower motor 320 may be coupled to the central shaft 221 of the second permanent magnet 220, and may rotate the second permanent magnet 220.

The housing drive part 400 may be located at a top of the first connection frame 120, and may be fixedly coupled to the first connection frame 120. The rotation shaft 410 of the housing drive part 400 may be provided in the third axis direction 13, and may pass through the first connection frame 120 so as to be coupled to the top of the housing 110. The housing drive part 400 may be driven to rotate the housing 110 about the third axis 13. According to an embodiment, the rotation shaft 410 of the housing drive part 400 may be located on the same line as the middle of the first permanent magnet 210 and the second permanent magnet 220 in the third axis direction 13. An electric motor may be used as the housing drive part 400.

The control part may control driving of the first lower motor 310, the second lower motor 320, and the housing drive part 400. The driving of the first lower motor 310 and the second lower motor 320 may be controlled to rotate the first permanent magnet 210 and the second permanent magnet 220 about the central shafts of the first permanent magnet 210 and the second permanent magnet 220, so that angles of the first permanent magnet 210 and the second permanent magnet 220 may be controlled. The driving of the housing drive part 400 may be controlled to rotate the housing 110 about the third axis 13. Through the control of the control part, a distribution of a magnetic field generated in the operation region T may be changed.

Hereinafter, a process of controlling a posture of the capsule endoscope 20 by using the magnetic field control device 10 described above will be described in detail.

The magnetic field control device 10 may be located outside a patient. When the patient swallows the capsule endoscope 20, it is difficult to accurately determine a position of the capsule endoscope 20 within a digestive organ. Therefore, a process of determining whether the capsule endoscope 20 is located close to or far away from the magnetic field control device 10 to set the position of the magnetic field control device 10 and aligning the permanent magnets 210 and 220 may be required.

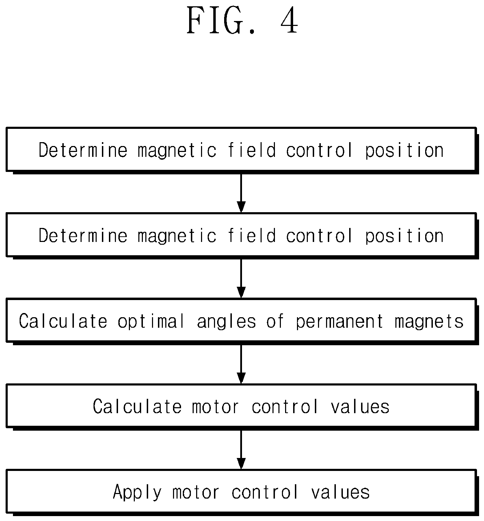

FIG. 4 is a flowchart showing a process of aligning magnet members according to an embodiment of the present invention.

Referring to FIG. 4, the control part may determine a magnetic field control position, and determine an alignment direction of the capsule endoscope 20. In addition, optimal angles of the permanent magnets 210 and 220 may be calculated so that a distribution of a magnetic field may be generated in the determined alignment direction of the capsule endoscope. The control part may calculate motor control values for aligning the permanent magnets 210 and 220 at the optimal angles, and apply the motor control values to the first lower motor 310 and the second lower motor 320, respectively. When angles of the permanent magnets 210 and 220 are changed, a magnetic field for controlling the posture of the capsule endoscope 20 may be formed in the operation region T.

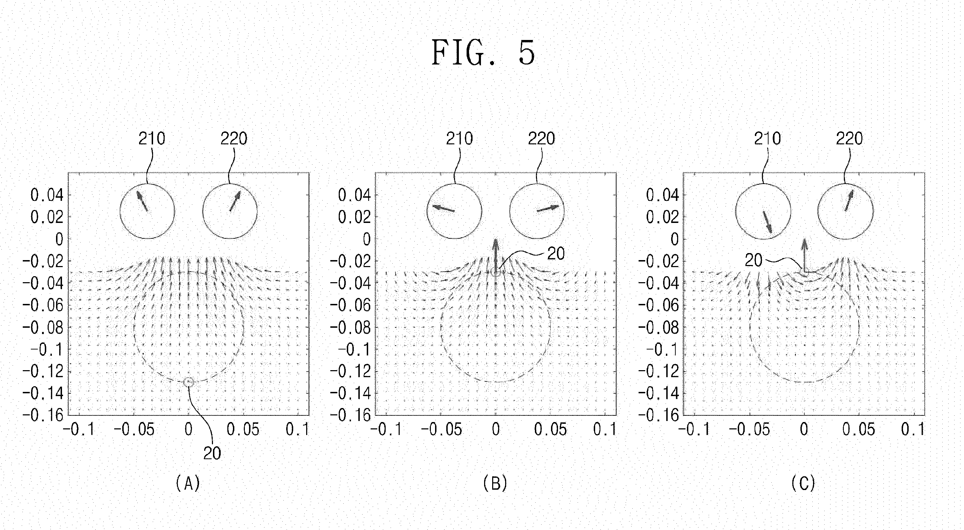

FIG. 5 is a view showing distributions of magnetic fields generated in an operation region due to various angle changes of the magnet members according to one embodiment of the present invention. Alignment angles of the magnet members were expressed in a counterclockwise direction based on the second axis direction 12, and the magnetic field and the magnetic force were expressed in the first to third axis directions 11 to 13 based on a point in which the capsule endoscope 20 was located.

First, referring to (A) of FIG. 5, when the first permanent magnet 210 is aligned at 117°, and the second permanent magnet 220 is aligned at 62.3°, it may be found that magnetic fields of 0 mT, 0 mT, and 10.8 mT and magnetic forces of 0 mN, 0 mN, and 36 mN are generated in the first to third axis directions 11 to 13 at the point in which the capsule endoscope 20 is located, respectively.

When the capsule endoscope 20 is located at a bottom of the operation region T having a diameter of about 10 cm, such as a stomach of the patient, as described above, the angles of the permanent magnets 210 and 220 may be controlled to generate a magnetic field and a magnetic force in the third axis direction 13 at the bottom of the operation region, and the magnetic field and the magnetic force may be used to control the posture of the capsule endoscope 20.

Referring to (B) of FIG. 5, when the first permanent magnet 210 is aligned at 165.9°, and the second permanent magnet 220 is aligned at 14.1°, it may be found that magnetic fields of 0 mT, 0 mT, and 123.6 mT and magnetic forces of 0 mN, 0 mN, and 879.4 mN are generated in the first to third axis directions 11 to 13 at the point in which the capsule endoscope 20 is located, respectively.

When the capsule endoscope 20 is located at a top of the operation region T, as described above, the angles of the permanent magnets 210 and 220 may be controlled to generate a magnetic field and a magnetic force having relatively high intensities in the third axis direction 13 at the top of the operation region T, and the magnetic field and the magnetic force may be used to control the posture of the capsule endoscope 20.

Referring to (C) of FIG. 5, when the first permanent magnet 210 is aligned at 289.6°, and the second permanent magnet 220 is aligned at 70.37°, it may be found that magnetic fields of 99.8 mT, 0 mT, and 0 mT and magnetic forces of 0 mN, 0 mN, and 905.5 N are generated in the first to third axis directions 11 to 13 at the point in which the capsule endoscope 20 is located, respectively.

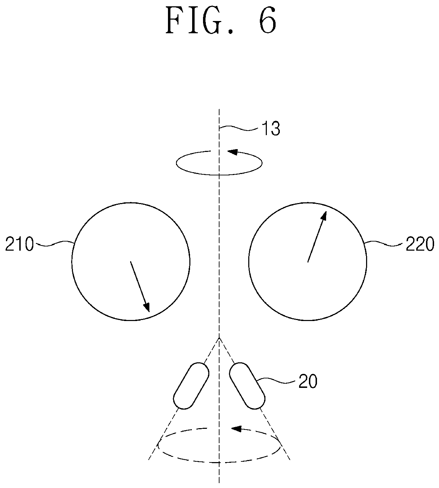

The angles of the permanent magnets 210 and 220 may be controlled as described above to control the posture of the capsule endoscope 20 such that the capsule endoscope 20 may be inclined at a predetermined angle with respect to the third axis direction 13. In this state, when the housing drive part 400 is driven to rotate the housing 110 about the third axis direction 13, as shown in FIG. 6, the capsule endoscope 20 may rotate about the third axis direction 13 while the position of the capsule endoscope 20 is fixed in the second axis direction 12.

Through the above control method, the magnetic field control device 10 may control the posture of the capsule endoscope 20 on a plane defined by the first and second axis directions 11 and 12, and may generate a rotational movement about the third axis direction 13 to generate a three-dimensional movement of the capsule endoscope 20. In this way, the magnetic field control device 10 may generate the three-dimensional movement of the capsule endoscope 20 while fixing the position of the capsule endoscope 20 on the plane defined by the first and second axis directions 11 and 12.

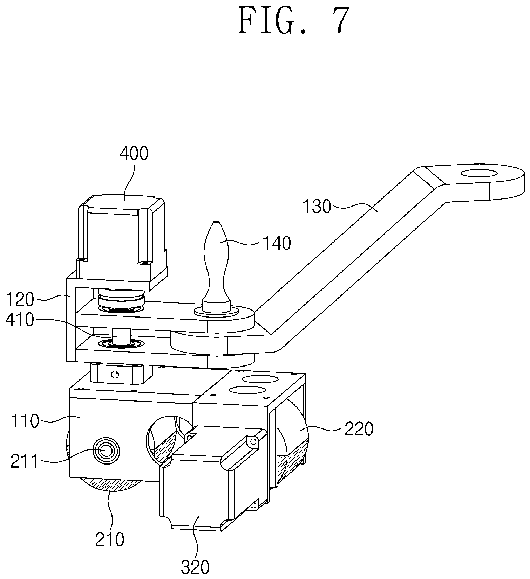

FIG. 7 is a view showing a magnetic field control device according to another embodiment of the present invention.

Referring to FIG. 7, unlike the embodiment of FIG. 1, the rotation shaft 410 of the housing drive part 400 may be located on the same line as a center of the first permanent magnet 210 or a center of the second permanent magnet 220 in the third axis direction 13. According to an embodiment, the rotation shaft 410 of the upper motor 400 may be located on the same line as the center of the first permanent magnet 210 in the third axis direction 13.

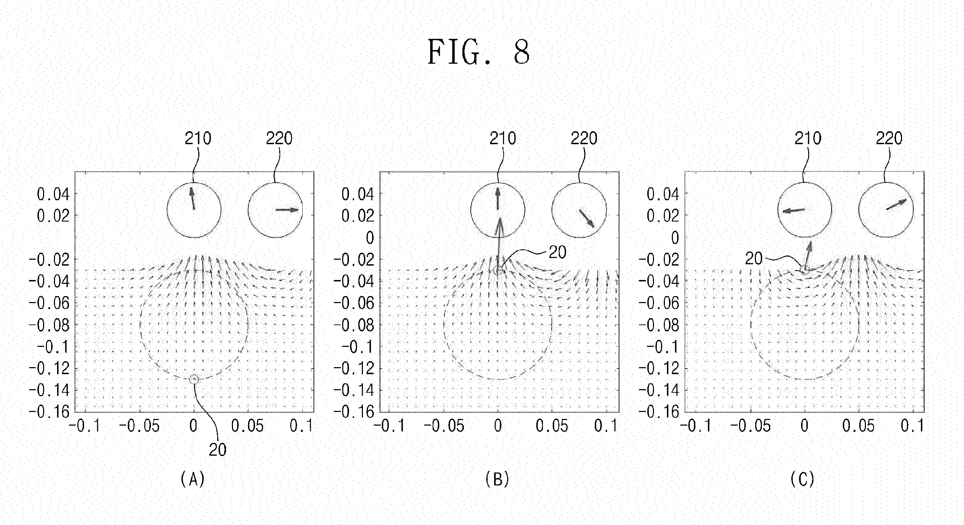

FIG. 8 is a view showing distributions of magnetic fields generated in an operation region due to various angle changes of magnet members according to another embodiment of the present invention.

Referring to (A) of FIG. 8, when the first permanent magnet 210 is aligned at 98.1°, and the second permanent magnet 220 is aligned at 0°, it may be found that magnetic fields of - 0.53 mT, 0 mT, and 8.63 mT and magnetic forces of 2.2 mN, 0 mN, and 30.3 mN are generated in the first to third axis directions 11 to 13 at the point in which the capsule endoscope 20 is located, respectively.

Referring to (B) of FIG. 8, when the first permanent magnet 210 is aligned at 90°, and the second permanent magnet 220 is aligned at 309.2°, it may be found that magnetic fields of -7.15 mT, 0 mT, and 148.4 mT and magnetic forces of 66.7 mN, 0 mN, and 1421.8.3 mN are generated in the first to third axis directions 11 to 13 at the point in which the capsule endoscope 20 is located, respectively.

Referring to (C) of FIG. 8, when the first permanent magnet 210 is aligned at 185.4°, and the second permanent magnet 220 is aligned at 26.44°, it may be found that magnetic fields of 88.74 mT, 0 mT, and 5.43 mT and magnetic forces of 175.6 mN, 0 mN, and 782.2 mN are generated in the first to third axis directions 11 to 13 at the point in which the capsule endoscope 20 is located, respectively.

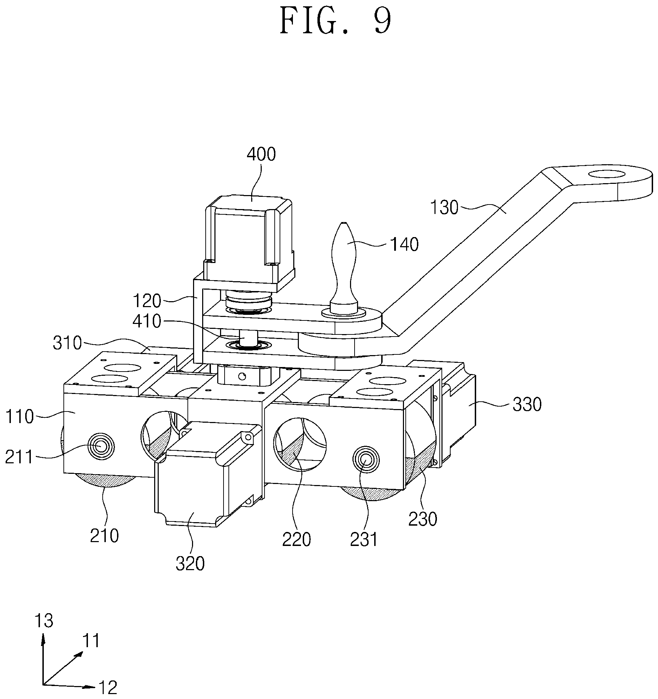

FIG. 9 is a perspective view showing a magnetic field control device according to still another embodiment of the present invention.

Referring to FIG. 9, magnetic field generation parts 210, 220, and 230 may include first to third magnet members. The first to third magnet members 210, 220, and 230 may be provided as permanent magnets. The first to third permanent magnets 210, 220, and 230 may be arranged sequentially in the second axis direction 12 at a predetermined interval, and arranged parallel to each other. According to one embodiment, the first to third permanent magnets 210, 220, and 230 may be arranged at the same height.

Magnet drive parts 310, 320, and 330 may include first to third lower motors. The first lower motor 310 may be coupled to the central shaft 211 of the first permanent magnet 210, and may rotate the first permanent magnet 210. The second lower motor 320 may be coupled to the central shaft 221 of the second permanent magnet 220, and may rotate the second permanent magnet 220. The third lower motor 330 may be coupled to a central shaft 231 of the third permanent magnet 230, and may rotate the third permanent magnet 230.

The control part may set a magnetic field control position, and set an alignment direction of the capsule endoscope 20. In addition, optimal angles of the permanent magnets 210, 220, and 230 may be calculated so that a distribution of a magnetic field may be generated in the set alignment direction of the capsule endoscope. The control part may calculate motor control values for aligning the permanent magnets 210, 220, and 230 at the optimal angles, and apply the motor control values to the first lower motor 310, the second lower motor 320, and the third lower motor 330, respectively. When angles of the permanent magnets 210, 220, and 230 are changed, a magnetic field for controlling the posture of the capsule endoscope 20 may be formed in the operation region T.

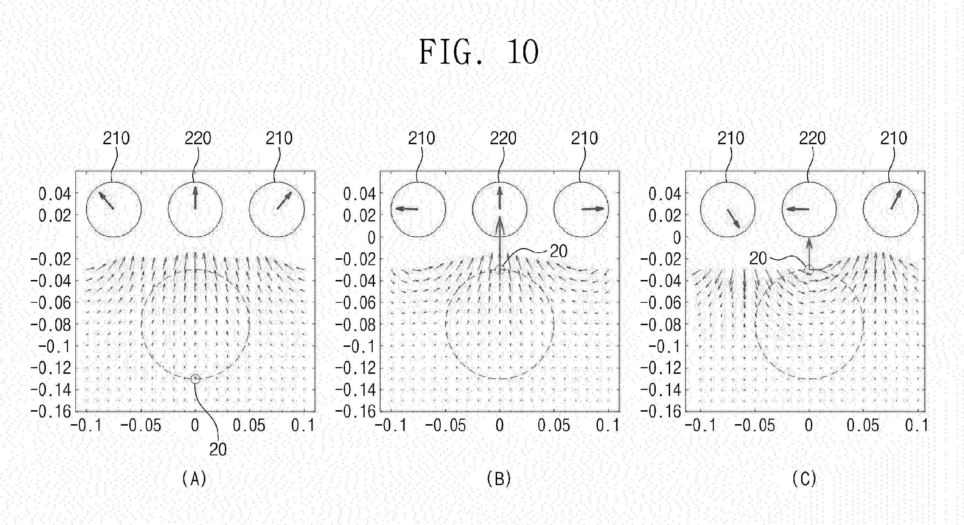

FIG. 10 is a view showing distributions of magnetic fields generated in an operation region due to various angle changes of magnet members according to the embodiment of FIG. 9.

First, referring to (A) of FIG. 10, when the first permanent magnet 210 is aligned at 144°, the second permanent magnet 220 is aligned at 90°, and the third permanent magnet 230 is aligned at 35.6°, it may be found that magnetic fields of 0 mT, 0 mT, and 14 mT and magnetic forces of 0 mN, 0 mN, and 44.2 mN are generated in the first to third axis directions 11 to 13 at the point in which the capsule endoscope 20 is located, respectively.

Referring to (B) of FIG. 10, when the first permanent magnet 210 is aligned at 230.8°, the second permanent magnet 220 is aligned at 89.9°, and the third permanent magnet 230 is aligned at 309.2°, it may be found that magnetic fields of 0 mT, 0 mT, and 160.6 mT and magnetic forces of 0 mN, 0 mN, and 1503.5 mN are generated in the first to third axis directions 11 to 13 at the point in which the capsule endoscope 20 is located, respectively.

Referring to (C) of FIG. 10, when the first permanent magnet 210 is aligned at 335.6°, the second permanent magnet 220 is aligned at 180°, and the third permanent magnet 230 is aligned at 24.4°, it may be found that magnetic fields of 109.1 mT, 0 mT, and 0 mT and magnetic forces of 0 mN, 0 mN, and 913.8 mN are generated in the first to third axis directions 11 to 13 at the point in which the capsule endoscope 20 is located, respectively.

The distribution of the magnetic field may be controlled as described above to control the posture of the capsule endoscope 20 when the capsule endoscope 20 is located at the top of the operation region T and when the capsule endoscope 20 is located at the bottom of the operation region T. In addition, the angles of the first to third permanent magnets may be controlled to control the posture of the capsule endoscope 20 such that the capsule endoscope 20 may be inclined at a predetermined angle with respect to the third axis direction 13. Further, when the housing drive part 400 is driven to rotate the housing 110 about the third axis direction 13, the capsule endoscope 20 may rotate about the third axis direction 13 while the position of the capsule endoscope 20 is fixed in the second axis direction 12.

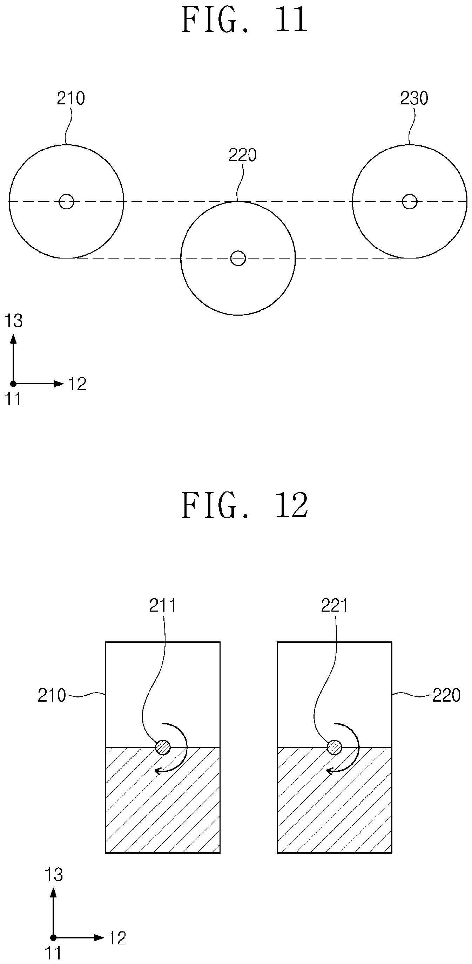

FIG. 11 is a view showing an arrangement of first to third magnet members according to yet another embodiment of the present invention.

Referring to FIG. 11, the first to third permanent magnets 210, 220, and 230 may be arranged at different heights. In detail, the second permanent magnet 220 may be disposed at a lower height than each of the first and third permanent magnets 210 and 230. Such an arrangement of the permanent magnets 210, 220, and 230 may reduce generation of a leakage magnetic field generated from the second permanent magnet 220 toward the first permanent magnet 210 or the third permanent magnet 230. In this state, when the capsule endoscope 20 is located under the second permanent magnet 220, the posture of the capsule endoscope 20 may be stably fixed in the third axis direction 13.

FIG. 12 is a view showing a magnetic field control device according to still yet another embodiment of the present invention.

Referring to FIG. 12, the first and second magnet members 210 and 220 may have arrangement directions that are different from the arrangement directions of the first and second magnet members described in FIG. 1. In detail, each of the first and second magnet members 210 and 220 may have a longitudinal direction arranged in the third axis direction 13. The central shafts 211 and 221 of the first and second magnet members 210 and 220 may be provided in a direction that is perpendicular to circumferential surfaces of the first and second magnet members 210 and 220, and may be located at 1/2 the heights of the first and second magnet members 210 and 220, respectively. Each of the first and second magnet members 210 and 220 may be bisected into upper and lower portions having different polarities based on the central shafts 211 and 221.

The first lower motor 310 may rotate the first magnet member 210 about the central shaft of the first magnet member 210, and the second lower motor 320 may rotate the second magnet member 220 about the central shaft of the second magnet member 220. Rotation angles of the first magnet member 210 and the second magnet member 220 may be controlled to change the distribution of the magnetic field generated in the operation region T.

Although two magnet members 210 and 220 have been illustrated in the present embodiment, the number of magnet members may be variously changed.

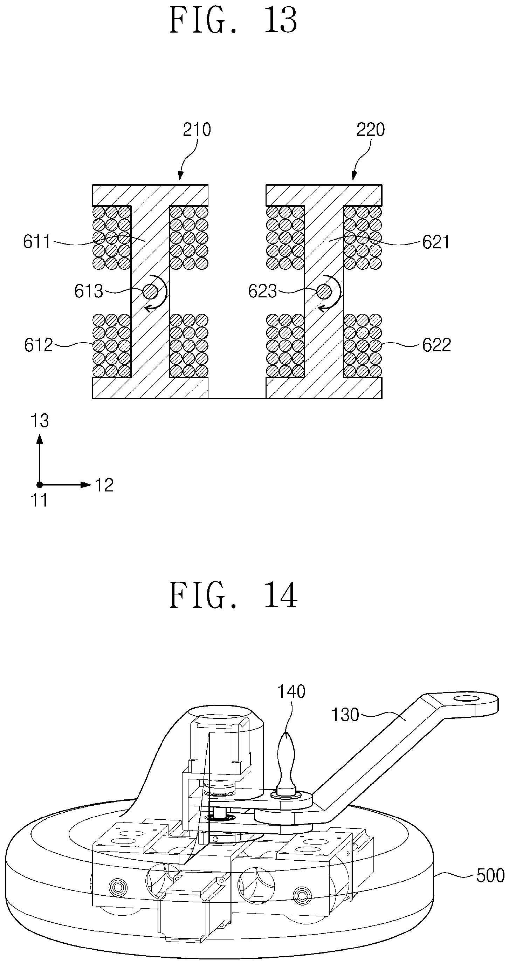

FIG. 13 is a view showing a magnetic field control device according to another embodiment of the present invention.

Referring to FIG. 13, the first and second magnet members 210 and 220 may be electromagnets. The first and second magnet members 210 and 220 may include cores 611 and 621, coils 612 and 622, and a power supply part (not shown).

Each of the cores 611 and 621 may have a cylindrical shape having a predetermined length, and may have a longitudinal direction arranged in the third axis direction 13. Central shafts 613 and 623 of the cores 611 and 621 may be provided in a direction that is perpendicular to circumferential surfaces of the cores 611 and 621, and may be arranged parallel to the first direction 11. The central shafts 613 and 623 of the cores 611 and 621 may be located at 1/2 the heights of the cores 611 and 621. At least two cores 611 and 621 may be provided, and may be spaced apart from each other in the second axis direction 12.

The coils 612 and 622 may be wound around the cores 611 and 621 by a predetermined number of turns, respectively, and the power supply part may apply a current to each of the coils 612 and 622.

The magnet drive parts 310 and 320 may rotate the cores 612 and 622 about the central shafts 613 and 623 of the cores 611 and 621.

When the control part performs control to apply the current to each of the coils 612 and 622, a magnetic field may be formed in the operation region T. When the control part changes an intensity of the current applied to each of the coils 612 and 622, a distribution of the magnetic field formed in the operation region T may be changed. In addition, when driving the magnet drive parts 310 and 320 are driven to rotate the cores 612 and 622 about the central shafts 613 and 623 of the cores 611 and 621 so as to change angles of the cores 611 and 621, the distribution of the magnetic field formed in the operation region T may be changed.

Although two magnet members 210 and 220 have been illustrated in the present embodiment, the number of magnet members may be variously changed.

FIG. 14 is a view showing a magnetic field control device according to still another embodiment of the present invention.

Referring to FIG. 14, the magnetic field control device 10 may further include a protective cover 500. The protective cover 500 may surround the housing 110, the magnet drive parts 310, 320, and 330, and the upper motor 400 so that the housing 110, the magnet drive parts 310, 320, and 330, and the upper motor 400 may not be exposed to an outside. The protective cover 500 may be formed of a non-magnetic material.

Although the exemplary embodiments of the present invention have been described in detail above, the scope of the present invention is not limited to a specific embodiment, and shall be interpreted by the appended claims. In addition, it is to be understood by a person having ordinary skill in the art that various changes and modifications can be made without departing from the scope of the present invention.

### [Industrial Applicability]

The magnetic field control device according to the present invention may be used to actively drive an object in which a permanent magnet is mounted, such as a magnetically driven capsule endoscope and a wired/wireless magnetic robot.

## Claims

1. A magnetic field control device comprising:
a housing having an accommodation space formed in the housing;
a first magnet member located in the accommodation space, having a central shaft arranged parallel to a first axis, and configured to generate a magnetic field;
a second magnet member located on one side of the first magnet member in a direction of a second axis that is perpendicular to the first axis, having a central shaft arranged parallel to the first axis, and configured to generate a magnetic field;
a magnet drive part for rotating the first magnet member and the second magnet member about the central shafts of the first magnet member and the second magnet member, respectively; and
a control part for controlling the magnet drive part to adjust angles of the first magnet member and the second magnet member.

2. The magnetic field control device of claim 1, wherein each of the first magnet member and the second magnet member is a permanent magnet bisected into an N-pole and an S-pole based on the central shafts of the first magnet member and the second magnet member.

3. The magnetic field control device of claim 2, wherein the permanent magnet has a cylindrical or spherical shape.

4. The magnetic field control device of claim 2, wherein the permanent magnet has a longitudinal direction arranged in a direction of the first axis.

5. The magnetic field control device of claim 2, wherein the permanent magnet has a longitudinal direction arranged in a direction of a third axis that is perpendicular to the first axis and the second axis.

6. The magnetic field control device of claim 1, wherein the magnetic fields generated from the first magnet member and the second magnet member, respectively, have a same intensity.

7. The magnetic field control device of claim 1, wherein each of the first magnet member and the second magnet member includes:
cores having a longitudinal direction arranged in a direction of a third axis that is perpendicular to the first axis and the second axis;
coils wound around the cores, respectively; and
a power supply part for applying currents to the coils, and
the control part controls the currents applied to the cores.

8. The magnetic field control device of claim 1, further comprising:
a third magnet member located on an opposite side of the second magnet member in the direction of the second axis, having a central shaft arranged parallel to the first axis, and configured to generate a magnetic field,
wherein the magnet drive part rotates the third magnet member about the central shaft of the third magnet member.

9. The magnetic field control device of claim 8, wherein the first to third magnet members are located at a same height.

10. The magnetic field control device of claim 8, wherein the first magnet member and the third magnet member are located at a higher position than the second magnet member in the direction of the third axis.

11. The magnetic field control device of claim 1, further comprising:
a housing drive part for rotating the housing about a third axis that is perpendicular to the first axis and the second axis.

12. The magnetic field control device of claim 11, further comprising:
a first connection frame fixedly coupled to an upper portion of the housing, and configured to support the housing drive part at a first point;
a second connection frame coupled to the first connection frame through a pin at a second point of the first connection frame; and
a handle coupled to the pin,
wherein the first connection frame is capable of performing relative rotation with respect to the second connection frame about the pin.
